# EUROPEAN PATENT APPLICATION

(11) **EP 2 777 717 A2**
(43) Date of publication of application: **17.09.2014**
(21) Application number: 14159884.7
(22) Date of filing: 14.03.2014
(51) Int. Cl.: A61L 2/24, A61L 9/00

(54) **Controlled and predictable 2-path-way decomposition of thermodynamically double unstable oxidising agents**

(30) Priority: 15.03.2013 US 201313839530
(71) Applicant: f-Oxyde GmbH, 2371 Hinterbrühl (AT)
(72) Inventor: Kedzierski, Alexandra, 2371 Hinterbrühl (AT); Thonhauser, Christian, 2371 Hinterbrühl (AT)
(74) Representative: KLIMENT & HENHAPEL

(57) **Abstract**

Disclosed herein is a method comprising: obtaining characteristics of a cleaning or disinfection application; querying a database comprising experimental relationships, theoretical relationships or both, between the characteristics of the cleaning or disinfection application and a composition; determining one or more compositions suitable for the cleaning or disinfection application based on the characteristics and the database.

## Description

### Background

Peroxo-compounds may be used for oxidation of organic material and associated disinfecting action, mainly in form of inorganic compounds (e.g., hydrogen peroxide and adducts, persulphates) or simple, water soluble organic peroxides (e.g. peracetic acid or 2-butanone peroxide).

These peroxo-compounds may be used at various pH and in multiple fields of use. They may be applied as pure substances or mixtures with acids, alkali, or alcohols. Cleaning and antimicrobial applications of peroxo-compounds may suffer from either too slow or too fast decomposition the peroxo-compounds as well as incompatibility with high pH environment or organic peroxides. Moreover, antimicrobial applications of peroxocompounds may not meet the requirements of highly challenging exposure scenarios (e.g., testing for long term exposure with public diseases).

### Brief Description of the Invention

Disclosed herein is a method comprising: obtaining characteristics of a cleaning or disinfection application; querying a database comprising experimental relationships, theoretical relationships or both, between the characteristics of the cleaning or disinfection application and a composition; determining one or more compositions suitable for the cleaning or disinfection application based on the characteristics and the database.

According to an embodiment, the characteristics are selected from the group consisting of: whether the cleaning or disinfection application includes disinfection, cleaning or both; composition of contaminant to be cleaned in the cleaning or disinfection application; surface from which the contaminant is to be cleaned the cleaning or disinfection application; whether infectants are inside living cells; whether the infectants are dispersed, or in a biofilm; desirability of surfactants in the one or more compositions; limitation on duration of disinfection; feasibility of rinsing; possible pH; scale of the cleaning or disinfection application; physical form of the one or more compositions; whether colour indication of organic matter is desired in the one or more composition; and a combination thereof.

According to an embodiment, the method further comprises determining conditions for the one or more composition, wherein the one or more compositions are applied under the conditions for the cleaning or disinfection application.

According to an embodiment, the one or more compositions and the conditions are selected from a group consisting of: pH; existence and composition of pH buffering substances; existence and composition of chelating agent; composition and concentration of catalysts; expected total decomposition kinetics of the one or more compositions; existence and composition of surfactant; existence and composition of colour indicator; cost; material compatibility; and a combination thereof.

According to an embodiment, the one or more compositions comprise a peroxo-compound.

According to an embodiment, the peroxo-compound if configured to undergo reductive decomposition and oxidative decomposition under a same condition.

According to an embodiment, the peroxo-compound releases oxygen-containing radicals upon reductive decomposition.

According to an embodiment, the peroxo-compound release O₂ upon oxidative decomposition.

According to an embodiment, the one or more compositions are configured to remove organic matters from a surface at essentially neutral pH.

According to an embodiment, the one or more compositions are configured to remove soiling form a surface at essentially neutral pH.

According to an embodiment, the one or more compositions comprise one or more catalysts configured to affect the reductive decomposition and the oxidative decomposition.

According to an embodiment, the one or more compositions comprise one or more catalysts are configured to affect ratio, amounts or both of oxygen-containing radicals and O₂ released upon decomposition of the peroxo-compound.

Also disclosed herein is a computer program product comprising a computer readable medium having instructions recorded thereon, the instructions when executed by a computer implementing any one of the methods disclosed herein.

Also disclosed herein a computer-implemented method, wherein the method is implemented in a computer system comprising one or more processors configured to execute one or more computer program modules, the method comprising: obtaining, on electronic storage media accessible to the one or more processors; executing, on the one or more processors of the computer system, one or more computer program modules configured to executing any one of the methods disclosed herein; and displaying, on an electronic display communicatively linked with the one or more processors of the computer system, the discrete pupil profile.

Also disclosed herein a composition comprising: a peroxo-compound configured to undergo both reductive decomposition and oxidative decomposition under a same condition; one or more catalysts configured to affect the reductive decomposition and the oxidative decomposition; wherein the composition are suitable for cleaning a surface, disinfecting a surface or both.

According to an embodiment, the peroxo-compound are configured to undergo both reductive decomposition and oxidative decomposition under a pH < 10.

According to an embodiment, the peroxo-compound releases oxygen-containing radicals upon reductive decomposition.

According to an embodiment, the peroxo-compound release O₂ upon oxidative decomposition.

According to an embodiment, the one or more catalysts are configured to affect ratio, amounts or both of oxygen-containing radicals and O₂ released upon decomposition of the peroxo-compound.

According to an embodiment, the surface is a surface of a semiconductor wafer.

### Brief Description Of Figures

Fig. 1 schematically shows a method according to an embodiment.
Fig. 2 shows two decomposition routes of hydrogen peroxide.
Fig. 3 shows both, the massive pH - dependency of catalyst performance towards peroxide decomposition (total of O₂ and oxygen-containing radicals considered) as well as the significantly higher performance at all pH of ruthenium(III) compared to ferric or manganese compounds.
Fig. 4 shows an indication with respect to O₂ and oxygen-containing radicals ratios that can be reached by single catalysts.
Figs. 5 and 6 show an example for mixed catalyst at three different pH values and numerous molar ratios between the two catalysts species.
Fig. 7 shows the concentration dependency of the colour change of the present composition upon contact with a carbohydrate.

### Detailed Description

The reason peroxo-compounds may not be optimal in performance in their cleaning and antimicrobial applications is that catalyst that catalyses the decomposition of the peroxo-compounds exhibits different efficacy with respect to different decomposition pathways of the peroxo-compounds. The term "peroxo-compound" as used herein means a compound that contains at least on peroxo group. When in an aqueous solution in presence of certain catalyst (e.g., Fe²⁺ or Ti³⁺), peroxo-compounds may exhibit double-instability, which means, both oxidation and reduction of the peroxo-compounds may occur (i.e., thermodynamically favourable) under the same condition. When the peroxo-compounds are oxidized (oxidative decomposition), oxygen gas (O₂) may be released; when the peroxo-compounds are reduced (reductive decomposition), oxygen-containing radicals such as HO (hydroxyl) and HOO (hydroperoxyl) may be released. A single catalyst at a given condition (e.g., a given pH) leads to an essentially constant ratio of the peroxo-compounds molecules that undergo oxidative decomposition and reductive decomposition, which makes adjusting the ratio of produced oxygen gas (O₂) and radicals difficult for customization for different applications.

The amount and ratio of O₂ gas and oxygen-containing radicals may have a major influence on peroxo-compounds performance in cleaning and disinfection applications, which may be characterized by organic matter removal or dissolution, and disinfection seffects against target organisms. O₂ gas may be released during decomposition of the peroxo-compounds in the form of microbubbles, which may mechanically break up solid infectant or contaminants and make oxygen-containing radicals better penetrate the solid infectant or contaminants. Oxygen-containing radicals released during decomposition of the peroxo-compounds chemically react with infectant or contaminants, in both solid or dispersed forms and effect mineralization of organic matter and genotoxic and/or mutagenic impact on organisms. For example, oxygen-containing radicals may attack microorganisms' internals while O₂ gas may ease radical induced condensation of certain types of polymeric organic substances (e.g. polyphenols). The amount and ratio of O₂ gas and oxygen-containing radicals from decomposition of peroxo-compounds may be affected by factors such as concentration of the peroxo-compounds, chemical composition of the solvent in which the peroxo-compounds are dispersed, pH, composition and concentration of catalyst, etc. The optimal amount and ratio of O₂ gas and oxygen-containing radicals from decomposition of peroxo-compounds may depend on the specification application. However, given a specification application (including e.g., the type, quantity, physical and chemical restraints, etc.), determining the optimal condition (e.g., pH, solvent, catalyst composition, concentration) for applying the peroxo-compounds in that specification application is not always straightforward. Such a determination allows control of the decomposition pathways of the peroxo-compounds and effective cleaning and/or disinfection.

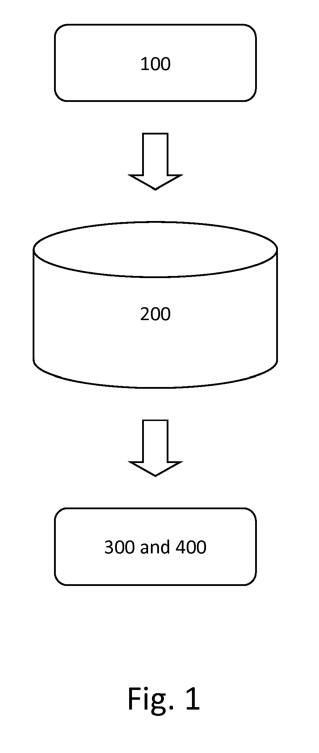

According to an embodiment, as schematically shown in Fig. 1, characteristics 100 of a cleaning or disinfection application are obtained; a database 200 may be queried, where the database 200 may include experimental and/or theoretical relationships between characteristics of a cleaning or disinfection application and a composition; compositions 300 and their conditions 400 suitable for the cleaning or disinfection application may be determined based on the characteristics 100 and the database 200. The characteristics 100 may include any combination of, but are not limited to:
- Disinfection, cleaning or both desired in the application?
- If cleaning needed, what is the contaminant to be cleaned?
- If cleaning needed, what is the surface from which the contaminant is to be cleaned?
- If disinfection needed, are the infectants inside living cells?
- If disinfection needed, are the infectants generally isolated / dispersed, or in a dense biofilm?
- Are surfactants desirable, possible or undesirable?
- If disinfection needed, is there any limitation on the duration of disinfection?
- If disinfection needed, is rinsing feasible or absolutely undesirable?
- What pH is possible?
- What is the scale of application?
- What is the desired physical form (e.g., solid or solution) of the composition?
- Is colour indication of organic matter desired?

The compositions 300 and their conditions 400 may include any combination of, but are not limited to:
- pH
- existence and composition of pH buffering substances
- existence and composition of chelating agent
- composition and concentration of catalysts
- expected total decomposition kinetics of the compositions
- existence and composition of surfactant
- existence and composition of colour indicator
- cost
- material compatibility

According to an embodiment, the relationships in the database 200 may include any combination of, but are not limited to the following:

Disinfection of surfaces loaded with live organisms but without particularly severe amounts of soiling or biofilms. This application tends to prefer (1) rapid formation (e.g., within 5 - 15 minutes) and essentially complete reductive decomposition of peroxo-compounds, which releases oxygen-containing radicals; (2) rinsing might be undesired; (3) a pH close to neutral or slightly acidic; (4) a concentration of 0.5 w/v % or less of the peroxo-compounds; (5) a surfactant is possible but not required (a foaming surfactant might increase the actual contact times on the surfaces if the surfaces are vertically positioned). The efficacy of the compositions 300 in the light of this application may be assessed by surface and/or suspension testing with standard bacteria or yeast.

Disinfection in protein / cell - matrices with organisms in presence of live cells. Oxidizing agent (formaldehyde is the reference substance in this context) alone may not be effective in this application. This application tends to prefer (1) longer exposure time to peroxo-compounds such as over 6 hours; (2) a concentration up to 5 w/v % of the peroxo-compounds; (3) both reductive and oxidative decomposition of the of the peroxo-compounds; (4) a pH close to neutral or unlikely to cause corrosion. This application covers epidemics control as required by several governments, for example in Germany the Robert-Koch-Institute listing.

Combined cleaning and disinfection of surfaces that might or might not be contaminated with living organisms but are dominated by a significant, in most cases even visible amount of organic soiling. This application tends to prefer (1) predominant oxidative decomposition of the of the peroxo-compounds, which release O₂ gas microbubbles; (2) a pH above 13 or low pH is possible but not required; (3) presence of surfactant; (4) presence of chelating agents; (5) if large amounts of organic material are expected to be removed, colour indication might become an option, which will help to identify not yet removed deposits.

Cleaning surfaces that have very little amounts of soiling but need to be free from any organic matter including greases. This application is often found in the semiconductor industry where semiconductor wafers are cleaned to remove organic matter. Semiconductor wafers are generally free of soiling but may have small amount of organic matter before cleaning. One method of cleaning such surfaces uses hydrogen peroxide under highly acidic conditions that induce rapid and complete decomposition of the peroxide. This method may be both costly and hazardous. The peroxo-compounds with the compositions 300 determined using the method described above may be equally effective but much less hazardous. This application tends to prefer (1) a pH close to neutral; (2) high ratio of oxygen-containing radicals to O₂ gas.

Substances that may be used in the compositions 300 may include, but not limited to:

Oxidizers or peroxo-compounds, such as inorganic peroxides that may exhibit thermodynamical double-instability in aqueous solution. If the inorganic peroxides are electrochemical potential determining in a composition, which is probable for most cases, chemicals that can be present in oxidized or reduced form (mainly transition metal catalysts and redox indicators) in the aqueous solution preferably do not exceed a concentration that will significantly change the electrochemical potential of the composition and thereby might lead to a situation where the peroxides cease to exhibit double-instability and therefor can only be decomposed reductively.

Oxidizers or peroxo-compounds may be liquid oxidizers such as hydrogen peroxide aqueous solution, peroxoacids or nonaromatic organic peroxides.

Oxidizers or peroxo-compounds may be solid oxidizers. Solid oxidizers suitable for use in the compositions 300 may be divided into two groups. The first and most likely to be used group are inorganic hydrogen peroxide adducts in the form of salt / electrolyte. Sodium percarbonate and sodium perborate are examples in the first group. The second group are inorganic oxidizers that contain peroxo groups but decompose rapidly under suitable pH and catalyst conditions into free hydrogen peroxide. Potassium monopersulphate is an example in the second group. The solid oxidizers may buffer pH in an aqueous solution.

pH - adjusting agents. Any acid or base can be used for reaching the desired pH. pH may change (usually drop) rapidly upon contact of the compositions 300 with organic matter. This is particularly relevant for applications that prefer an alkaline pH in order to get the desired ionization / cleaning effect and that will be most affected by the presence of organic soiling. In this case the addition of large amounts of pH - buffering substances might be preferred. An application where this is of major concern is the long exposure time epidemics control where the disinfectant will encounter massive organic soiling and has to maintain its intended O₂ / oxygen-containing radicals ratio over a long period of time.

Catalysts. Catalyst may include those that contain transition metal ions (e.g., d-elements, such as iron) that are able to exhibit several discrete oxidation states. Examples include ferric iron (from chloride sulphate) as well as iron - complex compounds like ferric EDTA or hexacyanoferrate (III). Manganese-containing chemicals are also a viable option with manganese dioxide being one example, which is an effective promoter of oxidative decomposition of peroxo-compounds. Another example is ruthenium (III) which bears the same electron configuration as ferric iron. Although being rare and expensive, the amounts needed are far less than comparable iron compounds and it is perfectly usable from both an economical and environmental/toxicological perspective. More widely used ruthenium chelates will fulfil the role as specific catalyst as well.

Redox indicator. Redox indicators can be added to compositions 300 as needed. Redox indicators will change colour upon either changes in overall electrochemical potential of the aqueous solution or by changes in availability of elementary oxygen dissolved into the solution. The colour change might be evaluated by simple instrumentation or visually. In both cases the comparison with the solution prior to contact with organic material is needed. For visual evaluation the situation can be eased by adding mixtures of redox indicators that will not only lose or gain colour intensity upon contact with organic matter but that will also provide an actual change in colour, which is easier to follow visually. Examples of redox indicators preferred in the electrochemical potential range established by hydrogen peroxide or hydrogen peroxide adducts are methylen blue, phenosafranin, ferroin and ferric EDTA. Ferric EDTA can therefor act as both colour indicator and catalyst and the occurrence of an intense violet colour in its intact form upon contact with peroxides makes this double use possible.

Other co-formulants. Certain substances that can be added to compositions 300 might enhance the cleaning / dissolution properties for organic soiling or the compatibility with hard water. For improving cleaning efficacy usually relatively oxidation resistant surfactants are added (e.g. sodium dodecylsulphate) or some builder substances might also support the removal of organic soiling. For stabilization of water hardness the choice of substances is relatively easy, since peroxygen does not destroy organic chelates as quickly as free chlorine does. Inorganic meta- or polyphosphates are suitable examples, but these compounds have to be used in large amounts and are therefore comparatively expensive. Organic chelates like the ethylene diamin acetic acid products and their similarly structured equivalents can be used but the potential and neither wanted nor required formation of peracetic acid upon action of peroxocompounds is preferably ruled out by selecting an appropriately high pH.

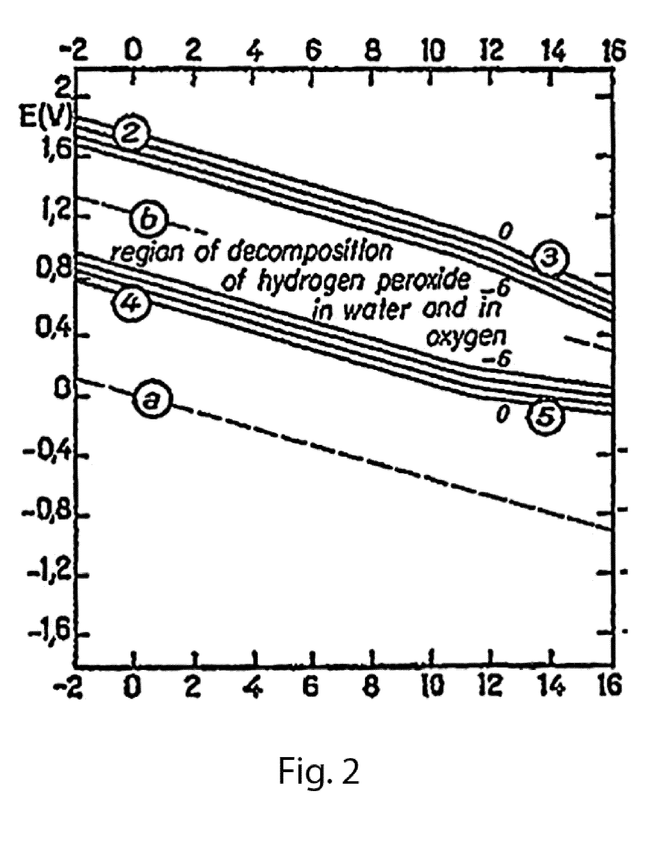

Hydrogen peroxide appears unstable and reducible to water below the family of lines (2-3) (Fig. 2); it appears unstable and oxidizable to oxygen above the family of lines (4-5) (Fig. 2). These two domains of instability have a common area, in which hydrogen peroxide is doubly unstable and can decompose into water and oxygen according to the reactions: H₂O₂+2H⁺+2e⁻→2H₂O and H₂O₂→O₂+2H⁺+2e⁻.

Fig. 2 shows that hydrogen peroxide or other sources of peroxo- groups exhibit decomposition both reductive and oxidatively.

Experimental data for effects of catalysts and mixtures with respect to O₂ and oxygen-containing radicals.

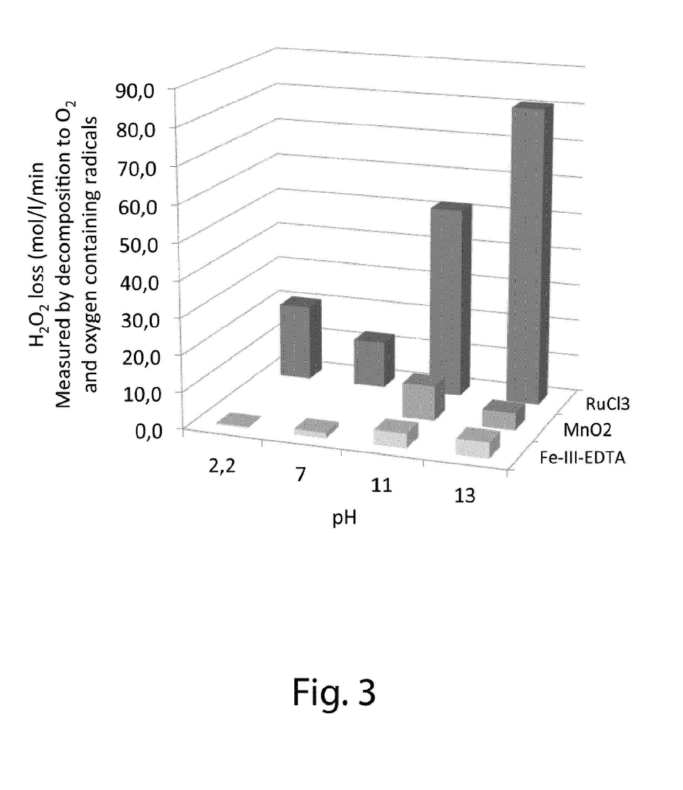

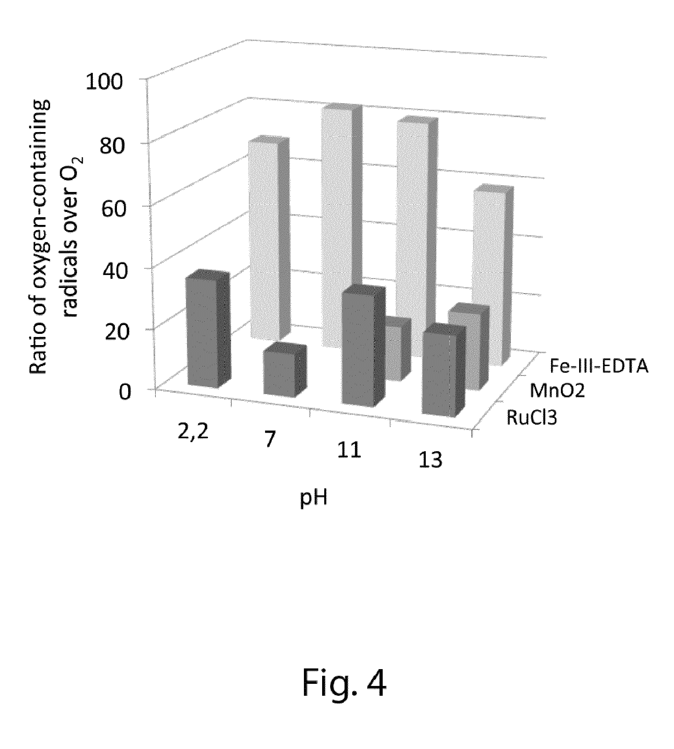

The ratio of O₂ and oxygen-containing radicals with a certain catalyst or mixtures of catalysts at a given pH and upon contact with an exemplary organic substance, mainly glycin, has been determined by overall loss in peroxide concentration (through decomposition to O₂ and oxygen-containing radicals) by cerium(IV) volumetric procedure and the recording of oxygen pressure arising from the same assay (O₂ only). Figs. 3 and 4 show a concise overview of catalysts at different pH values and their absolute performance in terms of total decomposition as well as their achieved ratio of O₂ and oxygen-containing radicals.

Fig. 3 shows both, the massive pH - dependency of catalyst performance towards peroxide decomposition (total of O₂ and oxygen-containing radicals considered) as well as the significantly higher performance at all pH of ruthenium(III) compared to ferric or manganese compounds.

Fig. 4 gives an indication with respect to O₂ and oxygen-containing radicals ratios that can be reached by single catalysts. Ferric EDTA almost fully suppresses the oxidative decomposition of peroxides and thereby will be the catalyst of choice when predominantly hydroxyl radical formation is beneficial for a given application. On the other hand, both manganese dioxide and ruthenium(III) give a much higher extent of elementary oxygen formation and therefore are more suitable for applications where the mechanical action of micro gas bubbles is supposed to promote the desired effects towards organic matter and / or microbiological contamination. The range observed here is about one order of magnitude, however, other data not displayed here suggest an even higher range of ratios of oxygen-containing radicals over O₂ between 5 and 300. This gives an opportunity for selecting catalysts and mixtures thereof that will achieve the desired effect.

### Performance of catalyst mixtures

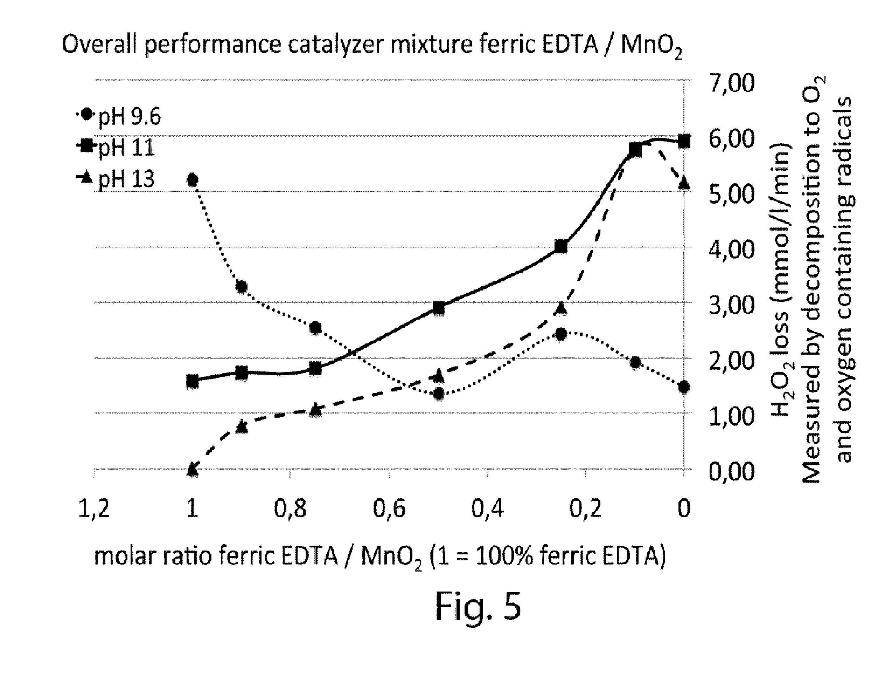

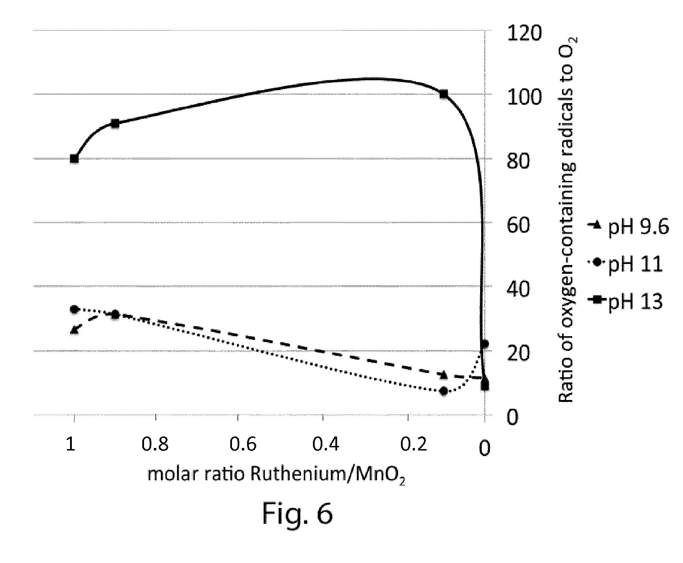

Figs. 5 and 6 show an example for mixed catalyst performance (in this case ferric EDTA and manganese dioxide) at three different pH values and numerous molar ratios between the two catalysts species. The pure catalysts (data point at 1 and 0 ratio) perform identical to the tests displayed in Fig. 3. More important a change in catalyst ratio will provide the desired shift in performance but without any significant deviations from a proportional behaviour. Therefore desktop-based calculation of overall catalyst performance (total decomposition of peroxide) is possible and will give reliable results.

In accordance with the findings for the pure catalysts manganese dioxide does not exhibit a particularly pronounced catalytic activity at lower pH, in this case 9.6. Therefor the O_{2/}oxygen-containing radicals ratio at this pH is solely dominated by the ruthenium(III) present and it is obviously independent from the catalyst concentration. At the higher pH values the formation of O₂ by manganese dioxide becomes more pronounced and in this case it is probably relatively difficult to find a combination of these two catalysts that will have a intermediate effect level on oxygen-containing radicals and O₂ formation, at least at the pH values tested. Other combinations of catalysts not displayed here work fine in this aspect and allow a predictable contribution of oxygen-containing radicals and O₂ to be realised at a given pH and with a peroxide decomposition rate that will exploit the oxidizer capacity over the exposure time envisaged.

### Composition examples and testing strategy

In order to be able to quantify the extent a desired effect towards organic matter removal / alteration or biocidal properties is achieved, we decided to use immersion testing of a dried protein / carbohydrate sample and microbiological efficacy testing according to the European Standard for Suspension Testing EN 1276 at exposure times between 5 and 60 minutes, present organic challenge and *Pseudomonas fluorescens* as test species that is hard to control by means of normal peroxo-compounds. The results were compared to data for an aqueous solution containing the pH - determining agent but nothing else (for organic matter decomposition quantification) and to biocidal products on the market that contain peroxo-compounds and also ethanol based disinfectants as reference biocides (EN 1276 suspension testing).

The dried organic matter was tested for an increase in flexural modulus, an increase in diameter by swelling as well as a change in colour by bleaching of the yellow yolk protein.

Efficacy results from EN 1276 were evaluated after calculation of the killing rate as - log CFU (colony forming units).

The present invention is further illustrated in the following preferred embodiments and examples without being restricted thereto.

### EMBODIMENTS OF THE INVENTION:

1. A method comprising:
   obtaining a characteristic of the system required for cleaning or disinfection of the system; and
   performing cleaning or disinfecting of the system using a composition suitable for cleaning or disinfecting of the system; wherein the composition is derived by a database comprising experimental relationships, theoretical relationships or both, between the characteristic and the composition.
2. The method of embodiment 1, wherein the characteristics are selected from the group consisting of:
   whether the cleaning or disinfection application includes disinfection, cleaning or both;
   composition of contaminant to be cleaned in the cleaning or disinfection application;
   surface from which the contaminant is to be cleaned the cleaning or disinfection application;
   whether infectants are inside living cells;
   whether the infectants are dispersed, or in a biofilm;
   desirability of surfactants in the one or more compositions;
   limitation on duration of disinfection;
   feasibility of rinsing;
   possible pH;
   scale of the cleaning or disinfection application;
   physical form of the one or more compositions;
   whether colour indication of organic matter is desired in the one or more composition; and
   a combination thereof.
3. The method of embodiment 1 or 2, further comprising determining conditions for the one or more composition, wherein the one or more compositions are applied under the conditions for the cleaning or disinfection application.
4. The method of embodiment 3, wherein the one or more compositions and the conditions are selected from a group consisting of:
   pH; existence and composition of pH buffering substances; existence and composition of chelating agent; composition and concentration of catalysts; expected total decomposition kinetics of the one or more compositions; existence and composition of surfactant; existence and composition of colour indicator; cost; material compatibility; and a combination thereof.
5. The method of any one of embodiments 1 to 4, wherein the one or more compositions comprise a peroxo-compound.
6. The method of embodiment 5, wherein the peroxo-compound if configured to undergo reductive decomposition and oxidative decomposition under a same condition.
7. The method of embodiment 5 or 6, wherein the peroxo-compound releases oxygen-containing radicals upon reductive decomposition.
8. The method of any one of embodiments 5 to 7, wherein the peroxo-compound release O₂ upon oxidative decomposition.
9. The method of any one of embodiments 1 to 8, wherein the one or more compositions are configured to remove organic matters from a surface at essentially neutral pH.
10. The method of any one of embodiments 1 to 9, wherein the one or more compositions are configured to remove soiling form a surface at essentially neutral pH.
11. The method of any one of embodiments 6 to 10, wherein the one or more compositions comprise one or more catalysts configured to affect the reductive decomposition and the oxidative decomposition.
12. The method of embodiment 11, wherein the one or more compositions comprise one or more catalysts are configured to affect ratio, amounts or both of oxygen-containing radicals and O₂ released upon decomposition of the peroxo-compound.
13. A computer program product comprising a computer readable medium having instructions recorded thereon, the instructions when executed by a computer implementing the method of any one of embodiments 1 to 12.
14. A computer-implemented method, wherein the method is implemented in a computer system comprising one or more processors configured to execute one or more computer program modules, the method comprising:
   obtaining, on electronic storage media accessible to the one or more processors;
   executing, on the one or more processors of the computer system, one or more computer program modules configured to executing the method of any one of embodiments 1 to 12; and
   displaying, on an electronic display communicatively linked with the one or more processors of the computer system, the discrete pupil profile.
15. A composition comprising:
   a peroxo-compound configured to undergo both reductive decomposition and oxidative decomposition under a same condition;
   one or more catalysts configured to affect the reductive decomposition and the oxidative decomposition;
   wherein the composition are suitable for cleaning a surface, disinfecting a surface or both.
16. The composition of embodiment 15, wherein the peroxo-compound are configured to undergo both reductive decomposition and oxidative decomposition under a pH < 10.
17. The composition of embodiment 15 or 16, wherein the peroxo-compound releases oxygen-containing radicals upon reductive decomposition.
18. The composition of any one of embodiments 15 to 17, wherein the peroxo-compound release O₂ upon oxidative decomposition.
19. The composition of any one of embodiments 15 to 18, wherein the one or more catalysts are configured to affect ratio, amounts or both of oxygen-containing radicals and O₂ released upon decomposition of the peroxo-compound.
20. The composition of any one of embodiments 15 to 19, wherein the surface is a surface of a semiconductor wafer.

### EXAMPLES:

### Example 1

Short term disinfectant with a pH not requiring a post rinse, solid powder product.

**Table 1**

| **Formulant** | **Function** | **preferable concentration in final solution** | **range concentration in final solution** | **Comment** |
|---|---|---|---|---|
| Sodium Percarbonate | Primary oxidizer | 0.4 % w/w | 0.2 - 0.6 % w/w | equivalent to ca. 0.13% H₂O₂ |
| Ferric EDTA | Catalyst for oxygen-containing radicals | 0.015 % w/w | 0.007 - 0.02 % w/w | |
| Potassiumhexac yanoferrate(III) | Catalyst for O₂ | 0.004 % w/w | 0.002 - 0.006 % w/w | |
| Ruthenium(III)c hloride | Catalyst for O₂ and oxygen-containing radicals | 0.0002 % w/w | 0.0002 - 0.0003 % w/w | |
| Sulfamic Acid | pH adjustment | 0.19 % w/w | 0.05 - 0.35 % w/w | final pH 9.6 |
| Sodium Dodecylsulphate | Surfactant | 0.0015 % w/w | 0.0007 - 0.0025 % w/w | |

A composition like that bears relatively high catalyst concentrations in order to have total peroxide decomposition rates within 30 minutes (average exposure time) of 40 % or higher. The also relatively high oxidizer content is required to allow oxidation of the organic soil challenge present in EN 1276 - efficacy tests (0.3 g/L albumin). The traces of surfactant present support the accessibility of the aqueous solution towards the target cells. When reaching crevices on the target cells the intended contribution from O₂ will help in destroying cell membranes and thereby ease the access to the cell genome for the radicals formed by oxygen-containing radicals decomposition pathway.

### Example 2

Long term disinfectant for the German RKI epidemic control testing at exposure times up to 6 hours.

**Table 2**

| **Formulant** | **Function** | **Concentration in final solution** | **range concentration in final solution** | **Comment** |
|---|---|---|---|---|
| Sodium Percarbonate | Primary oxidizer | 1.6 % w/w | 0.8-2.5%w/w | equivalent to ca. 0.13% H₂O₂ |
| Ferric EDTA | Catalyst for oxygen-containing radicals | 0.010 % w/w | 0.005 - 0.015 % w/w | |
| Potassiumhexac yanoferrate(III) | Catalyst for O₂ | 0.002 % w/w | 0.001 - 0.003 % w/w | |
| Ruthenium(III)c hloride | Catalyst for oxygen-containing radicals and O₂ | 0.0001 % w/w | 0.00005 - 0.00015 % w/w | |
| Sulfamic Acid | pH adjustment | 0.8 % w/w | 0.03 - 0.2 % w/w | final pH ca. 9 |
| Sodium Dodecylsulphate | Surfactant | 0.0015 % w/w | 0.001 - 0.0025 % w/w | |
| Sodium Hydrogencarbon ate | additional buffer substance | 0.5 % w/w | 0.4 - 1 % w/w | maintainance of required pH over exposure times up to 6 hours upon contact with large amounts of organic substrate |

Long exposure times and very resistant target organisms, maybe even located inside of host cells (viruses) require the availability of the oxygen species formed from the peroxide over most of the contact time. Therefore the oxidizer content is massively increased compared to Example 1 and catalysts are present to a lesser extent. The long contact time and the much higher and more complex organic challenge compared to the EN 1276 test requires a stable pH over the exposure time. Therefore a pH buffer substance has to be added in order to capture acid formed by the oxygen-containing radicals decomposition as well as acid removed / OH⁻ formed by O₂ oxidative decomposition of the peroxide. All this results in a total concentration in the solution used for epidemic control that is around 3 %, something that is common for this particular use.

### Example 3

Combined cleaning and disinfecting agent for various organic matrices at a pH of > 10 and with integrated colour indication of organic matter present.

**Table 3**

| **Formulant** | **Function** | **Concentration in final solution** | **range concentration in final solution** | **Comment** |
|---|---|---|---|---|
| Sodium Percarbonate | Primary oxidizer | 0.4 % w/w | 0.2 - 0.6 % w/w | equivalent to ca. 0.13% H₂O₂ |
| Ferric EDTA | Catalyst for oxygen-containing radicals / Redox indicator | 0.0005 % w/w | 0.0003 - 0.0008 w/w | |
| Ruthenium(III)c hloride | Catalyst for oxygen-containing radicals and O₂ | 0.001 % w/w | 0.0005 - 0.0015 % w/w | |
| Caustic soda | pH adjustment | 0.025 % w/w | 0.01 - 0.04 % w/w | final pH 10 to 10.5 |
| Sodium EDTA | Water hardness stabilization | 0.075 % w/w | 0.05 - 0.09 % w/w | |
| Methylene blue | Redox indicator | 0.01 % w/w | 0.008 - 0.015 % w/w | |

The above composition targets mainly on organic matter removal from soiled surfaces and therefore contains a higher amount of ruthenium(III) that will act as both oxygen-containing radicals but also O₂ catalyst and thereby help the mechanical removal of tense organic soiling. Generally, a higher pH that leads to ionization of organic matter is preferable for this type of application, but due to the careful selection of identity and concentration of catalysts much lower pH values with better material compatibility and lesser requirements regarding post rinsing can be used. Products giving similar cleaning results usually have pH values between 12 and 14 and therefore require caustic agents in concentrations of 10 to 100fold as in the present composition. A colour change from pure, short wavelength blue to green shades can be visually (or instrumentally) observed upon reaction with significant amounts of organic matter ( > 100 mg/L of e.g. carbohydrates can be detected visually when compared to a sample of the solution that has not been in contact with organic matter).

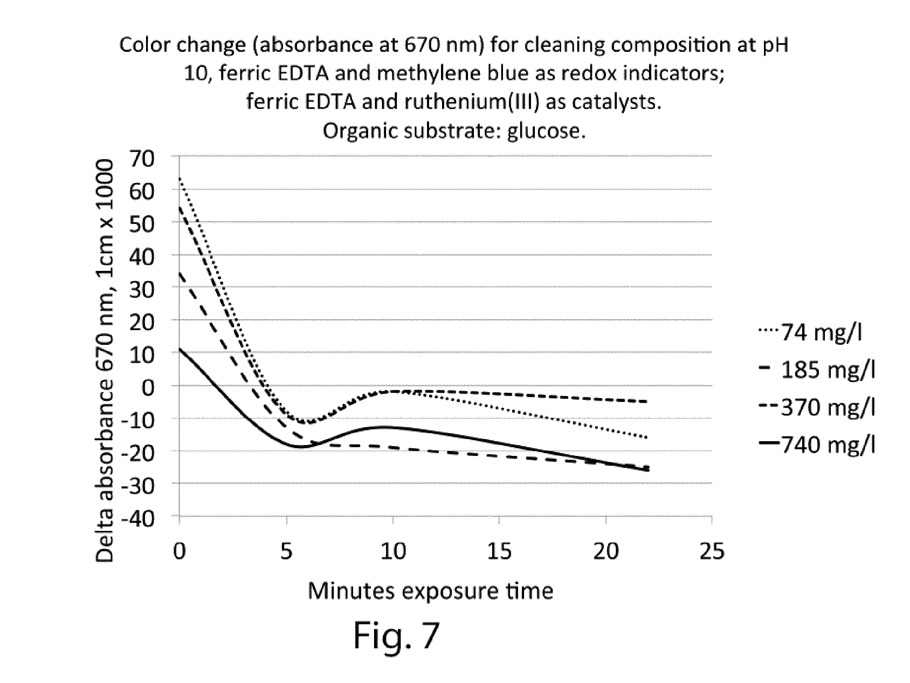

Fig. 7 shows the concentration dependency of the colour change of the present composition upon contact with a carbohydrate. Since it was measured at one wavelength only, the diagram does not give a very good indication of the actual and visible colour change from blue into turquoise / green that is taking place. The violet shade from the ferric EDTA significantly promotes the absorbance at 670 nm whereas the reduced form of ferric EDTA is yellowish enhancing the greenish appearance. The amount of colour change expressed in absorbance units is a function of the concentration of organic matter but there is so far no prove of exact linearity.

### Example 4

Oxidative cleaning agent for removal of traces of hard-to-oxidize organic matter from smooth, inorganic surfaces.

**Table 4**

| | **Formulant** | **Function** | **Concentration in final solution** | **Comment** |
|---|---|---|---|---|
| Component A | Hydrogen Peroxide 35% | primary oxidizer | 0.5 % as H₂O₂ | |
| Component B | Ferric EDTA | Catalyst for oxygen-containing radicals | 0.03 % | |
| | Potassiumhexac yanoferrate(III) | Catalyst for O₂ | 0.0005 % | |
| | Sulfamic Acid | pH adjustment | 0.05 % | pH between 3 and 4 to be reached, depending on water quality |

An oxidative cleaning agent of this composition will form predominantly high potential radical species. A minor contribution of elementary oxygen bubbles from reduction will help micro-convection of the agent on the surfaces to be cleaned. The overall catalyst concentration will deliver peroxide decomposition products at ambient temperatures over a period of 20 to 60 minutes.

### Results Example 1 - 3

**Table 5**

| | | | | **60 min exposure time, ambient temperature** | | | |
|---|---|---|---|---|---|---|---|
| | **-log CFU *P.fluor escens*** | **reference disinfec tant* - log CFU *P*. *fluorescens*** | **exposure time at ambient temperature** | **flexural modulus [ ]** | **reference substance NaOH at equivalent pH** | **swelling in percent of dry material** | **reference substance NaOH at equivalent pH** |
| Example 1 | 4 | 2.8 | 5 | 0.13 | 0.04 | 33 % | 18 % |
| Example 2 | 3.1 | 2.9 | 90 | 0.07 | 0.06 | 31 % | 21 % |
| Example 3 | 3.7 | 2.8 | 5 | 0.17 | 0.1 | 40 % | 25 % |

* BODE Chemie Bacillol 25 (10% ethanol, 9% iso-propanol, 6% n-propanol), applied undiluted, BODE CHEMIE HAMBURG, Germany

As can be seen from the above table, compositions according to examples 1 - 3 outperform a alcohol - based reference product both at long and short exposure times at EN 1276 suspension testing. Regarding the mechanical / cleaning testing parameters, all examples outperform an aqueous solution with similar pH with respect to both material swelling (and also discolourization of yellow yolk dye not displayed here) and flexural modulus (higher values are for more flexible material, maximum is 1.0)

While the invention is described and illustrated here in the context of a limited number of embodiments, the invention may be embodied in many forms without departing from the spirit of the essential characteristics of the invention. The illustrated and described embodiments, including what is described in the abstract of the disclosure, are therefore to be considered in all respects as illustrative and not restrictive. The scope of the invention is indicated by the appended claims rather than by the foregoing description, and all changes that come within the meaning and range of equivalency of the claims are intended to be embraced therein.

## Claims

1. A method comprising:
obtaining a characteristic of the system required for cleaning or disinfection of the system; and
performing cleaning or disinfecting of the system using a composition suitable for cleaning or disinfecting of the system; wherein the composition is derived by a database comprising experimental relationships, theoretical relationships or both, between the characteristic and the composition.

2. The method of Claim 1, wherein the characteristics are selected from the group consisting of:
whether the cleaning or disinfection application includes disinfection, cleaning or both;
composition of contaminant to be cleaned in the cleaning or disinfection application;
surface from which the contaminant is to be cleaned the cleaning or disinfection application;
whether infectants are inside living cells;
whether the infectants are dispersed, or in a biofilm;
desirability of surfactants in the one or more compositions;
limitation on duration of disinfection;
feasibility of rinsing;
possible pH;
scale of the cleaning or disinfection application;
physical form of the one or more compositions;
whether colour indication of organic matter is desired in the one or more composition; and
a combination thereof.

3. The method of Claim 1 or 2, further comprising determining conditions for the one or more composition, wherein the one or more compositions are applied under the conditions for the cleaning or disinfection application.

4. The method of claim 3, wherein the one or more compositions and the conditions are selected from a group consisting of:
pH;
existence and composition of pH buffering substances;
existence and composition of chelating agent;
composition and concentration of catalysts;
expected total decomposition kinetics of the one or more compositions;
existence and composition of surfactant;
existence and composition of colour indicator;
cost;
material compatibility; and
a combination thereof.

5. The method of any one of Claims 1 to 4, wherein the one or more compositions comprise a peroxo-compound.

6. The method of Claim 5, wherein the peroxo-compound if configured to undergo reductive decomposition and oxidative decomposition under a same condition.

7. The method of Claim 6, wherein the peroxo-compound releases oxygen-containing radicals upon reductive decomposition.

8. The method of Claim 6 or 7, wherein the one or more compositions comprise one or more catalysts configured to affect the reductive decomposition and the oxidative decomposition.

9. The method of Claim 8, wherein the one or more compositions comprise one or more catalysts are configured to affect ratio, amounts or both of oxygen-containing radicals and O₂ released upon decomposition of the peroxo-compound.

10. A computer program product comprising a computer readable medium having instructions recorded thereon, the instructions when executed by a computer implementing the method of any one of Claims 1 to 9.

11. A computer-implemented method, wherein the method is implemented in a computer system comprising one or more processors configured to execute one or more computer program modules, the method comprising:
obtaining, on electronic storage media accessible to the one or more processors;
executing, on the one or more processors of the computer system, one or more computer program modules configured to executing the method of any one of Claims 1 to 9; and
displaying, on an electronic display communicatively linked with the one or more processors of the computer system, the discrete pupil profile.

12. A composition comprising:
a peroxo-compound configured to undergo both reductive decomposition and oxidative decomposition under a same condition;
one or more catalysts configured to affect the reductive decomposition and the oxidative decomposition;
wherein the composition are suitable for cleaning a surface, disinfecting a surface or both.

13. The composition of Claim 12, wherein the peroxo-compound are configured to undergo both reductive decomposition and oxidative decomposition under a pH < 10.

14. The composition of Claim 12 or 13, wherein the peroxo-compound releases oxygen-containing radicals upon reductive decomposition.

15. The composition of any one of Claims 12 to 14, wherein the one or more catalysts are configured to affect ratio, amounts or both of oxygen-containing radicals and O₂ released upon decomposition of the peroxo-compound.
